# EUROPÄISCHE PATENTSCHRIFT

(11) **EP 2 460 531 B1**
(45) Veröffentlichungstag und Bekanntmachung des Hinweises auf die Patenterteilung: **04.06.2014**
(21) Anmeldenummer: 10193408.1
(22) Anmeldetag: 02.12.2010
(51) Int. Cl.: A61K 38/39, A61P 17/06

(54) **Collagen zur Verwendung in der Behandlung von Hauterkrankungen**
Collagen for use in the treatment of skin ailments
Collagène pour l'utilisation dans le traitement de maladies de la peau

(43) Veröffentlichungstag der Anmeldung: 06.06.2012
(73) Patentinhaber: MedSkin Solutions Dr. Suwelack AG, 48727 Billerbeck (DE)
(72) Erfinder: Kassner, Anja., 48161 Münster (DE); Trautmann, Martin., 48249 Dülmen (DE); Gütt, Sabine., 22559 Hamburg (DE)
(74) Vertreter: Gille Hrabal

(56) Entgegenhaltungen:
- WO-A2-01/64046
- US-A1- 2003 203 008

## Beschreibung

### EINLEITUNG:

Die vorliegende Erfindung betrifft tierisches Collagen zur Verwendung in der Behandlung entzündlicher und degenerativer Hauterkrankungen und damit verbundener Hautschädigungen wie insbesondere in der Behandlung von Psoriasis, Dermatitis, Neurodermitis, Rosacea, Urticaria (Nesselsucht), Pruritus (Juckreiz), Hautekzemen und aktinischer Keratose. Insbesondere betrifft die Erfindung gefriergetrocknetes Collagen in Form von schichtförmigen Auflagen, Sheets, Pads oder Masken zur vorgenannten Verwendung.

### HINTERGRUND:

Collagen ist ein bio-degradierbares, biokompatibles Protein, welches in der medizinischen Anwendung breite Verwendung findet, beispielsweise als Wundheilungsmittel, Trägermaterial in Wundauflagen oder als Hämostyptikum.

Auch in der Kosmetik ist die Verwendung von Collagen, beispielsweise als feuchtigkeitsregulierender Wirk- oder Hilfsstoff oder als Trägermaterial für kosmetische Auflagen verbreitet.

Insbesondere bei der kosmetischen oder therapeutischen Verwendung von Collagen als Trägermaterial in der topischen oder dermalen Anwendung werden in der Regel in solche Trägermaterialien kosmetische oder pharmazeutische Wirk-, Nähr- und/oder Pflegestoffe zur Auf- bzw. Einbringung auf die zu behandelnden Hautareale eingearbeitet. Derartige Wirkstoff-dotierte Trägermaterialien werden üblicherweise als Drug-Delivery-Formulierungen bezeichnet, woraus die eingebrachten Wirk- oder Aktivstoffe auf der behandelten Hautregion freigesetzt werden und dadurch eine Pflege- oder Heilwirkung im Sinne einer kosmetischen Pflegebehandlung oder einer therapeutische Behandlung bewirken.

Überraschend wurde gefunden, dass darüber hinaus tierisches Collagen selbst eine vorteilhafte Wirkung in der medizinischen Hautbehandlung, speziell in der Behandlung entzündlicher Hauterkrankungen, degenerativer Hauterkrankungen sowie der damit einhergehenden Hautschädigungen besitzt, Insbesondere zeigten sich die vorteilhafte Wirkung in der Behandlung von Hauterkrankungen aus der Gruppe die Psoriasis, Dermatitis, Neurodermitis, Rosacea, Urticaria (Nesselsucht), Pruritus (Juckreiz), Hautekzeme und aktinische Keratose umfasst, Desweiteren wurde gefunden, dass insbesondere die Verwendung von Collagen in Form gefriergetrockneter Auflagen, Sheets oder Masken, die zur Applikation mit einer geeigneten Aktivatorlösung rehydratisiert bzw. befeuchtet werden, zur Behandlung in den vorstehend genannten medizinischen Indikationen geeignet ist.

### STAND DER TECHNIK:

Üblicherweise werden obengenannte Hauterkrankungen durch Aufbringen pharmazeutischer Wirkstoffe wie insbesondere Cortison, Schmerzmittel, Antihistaminika, Neuroleptika u.a. in Form von Salben, Cremes, Gelen, Lotionen oder Schütteltinkturen behandelt. Auch die Behandlung mittels Bädern, fett-feuchten oder mit schwarzem Tee getränkten Umschlägen oder sogenannte PUVA-Anwendung (Psoralen plus UV-A), einer Kombinationstherapie aus Psoralen-Extrakt und UV-Licht, ist verbreitet. Darüber hinaus ist es bekannt, beispielsweise aus der WO 2007/076852 A1 oder der US 6,830,758 B2, Hauterkrankungen wie Psoriasis, Dermatitis oder Ekzeme mittels selbstklebenden okklusiven oder semi-okklusiven Verbänden oder Pflasterabdeckungen zu behandeln.

Der Nachteil solcher herkömmlicher Behandlungsmethoden mittels pharmazeutischer Wirkstoffe liegt insbesondere in den meist nicht unerheblichen Nebenwirkungen, die häufig auch anhaltende Langzeitschädigungen mit sich bringen. Die bekannten Bäder-, Umschlag- oder PUVA-Anwendungen sind insofern als nachteilig einzustufen, als diese meistens nur sehr geringe Behandlungserfolge mit sich führen und ggf. sehr aufwändig in der Applikation (Bäder, PUVA) sind. Der Nachteil der bekannten selbstklebenden okklusiven oder semi-okklusiven Verbände oder Pflasterabdeckungen, beispielsweise nach der WO 2007/076852 A1 oder der US 6,830,758 B2, ist insbesondere darin zu sehen, dass diese in der Regel aus synthetischen Matrixmaterialien hergestellt werden oder synthetische Kleber beispielsweise auf Basis von Polyacrylaten enthalten. Solche synthetischen Polymermatrices uns synthetischen Klebermaterialien sind häufig kritisch hinsichtlich der biologischen Verträglichkeit. Darüber hinaus müssen solche Abdeckungen in der Regel über einen Zeitraum von mehreren Stunden, mindestens einer Stunde, angewendet werden und zeigen Wirkeffekte zumeist erst nach mehreren Tagen der regelmäßigen Anwendung. Darüber hinaus werden solche Abdeckungen meist mit Wirkstoffen dotiert und damit primär als Trägermaterial zur Applikation der Wirkstoffe auf den erkrankten Hautarealen verwendet.

Insbesondere in Hinblick auf solche selbstklebenden okklusiven oder semi-okklusiven Abdeckungen sind die erfindungsgemäßen gefriergetrockneten Collagenzubereitungen in Form schichtförmiger Pads, Auflagen oder Masken vorteilhaft in der Behandlung von Hauterkrankungen wie Psoriasis, Dermatitis, Neurodermitis, Rosacea, Urticaria (Nesselsucht), Pruritus (Juckreiz), Hautekzemen und aktinischer Keratose, da diese einerseits aus einem hochverträglichen biologischen Material bestehen und außerdem bereits nach deutlich kürzerer Auflagezeit und in der Regel bereits unmittelbar bei der ersten Behandlung eine Wirkung zeigen, indem sie die entsprechenden Symptome der Hauterkrankungen deutlich lindern.

Aus der US 2003/032601 ist eine Methode zur Isolierung von Collagen aus marinen Schwämmen zur Herstellung von Nano- und Mikropartikeln aus Schwamm-Collagen sowie deren Verwendung in üblichen pharmazeutischen halb-festen oder flüssigen Formulierungen wie Cremes oder Gelen zur Behandlung von Cyclooxygenase-abhängigen entzündlichen Erkrankungen, degenerativen Hauterkrankungen oder Hautschädigungen, z. B. Erythema nach UV-Strahlung und Verletzungen sowie von Psoriasis oder Dermatitis bekannt. Außerdem wird darin offenbart, dass die nanopartikulären Schwamm-Collagenpartikel, die auch in gefriergetrockneter Form vorliegen können, als Cyclooxygenase-Inhibitoren wirken und einen antientzündlichen Effekt, speziell bei Cyclooxygenase-abhängigen entzündlichen Erkrankungen, sowie einen antioxidativen Effekt besitzen. Ein entsprechender Effekt für Collagen allgemein und somit insbesondere auch für tierisches Collagen wird hierin nicht beschrieben, vielmehr wird tierisches Collagen explizit als nachteilig genannt. Auch werden keine Applikationsformen des marinen Schwammcollagens in Form von Auflagen, Pads oder ähnlichen schichtförmigen festen Applikationsformen beschrieben. Solche schichtförmigen festen Applikationsformen von Collagen zeichnen sich gegenüber halbfesten, flüssigen bzw. Creme-, Gel- oder Lotionförmigen Formulierungen, die Schwammcollagen-Nanopartikel quasi als Wirkstoffbestandteil enthalten, dadurch aus, dass das Collagen als Wirksubstanz hochkonzentriert, quasi hochrein, appliziert werden kann.

Daneben existiert umfangreicher Stand der Technik zu kosmetischen oder pharmazeutischen Hautbehandlungsmitteln, worin Collagen entweder als ein mögliches Trägermaterial eingesetzt wird, ohne dass für dieses eine konkrete Wirkung in den erfindungsgemäßen Indikationen beschrieben wird, oder Collagen wird als einer unter zahlreichen möglichen Wirk- oder Hilfsstoffen in kosmetischen oder pharmazeutischen Hautbehandlungsformulierungen genannt, wiederum ohne konkret hierfür eine vorteilhafte Wirkung in einer der erfindungsgemäßen Indikationen zu offenbaren. Beispielsweise können die in der oben genannten WO 2007/076852 beschriebenen semi-okklusiven Pflasterzubereitungen auch Hilfsstoffe zur Rückfettung und Pflege enthalten, die unter anderem pflanzliche und tierische Extrakte und Öle wie z.B. Collagen umfassen. Eine besondere Wirkung des Collagens selbst, insbesondere in Form gefriergetrockneter Auflagen, speziell in der Behandlung irritierter Haut und Hauterkrankungen gemäß der vorliegenden Erfindung ergibt sich auch daraus nicht.

Aus dem Bereich der schichtförmigen Collagenmatrices zur Verwendung in der Hautbehandlung sind aus der DE 4028622 A1 kosmetische Collagenschwämme z.B. in Form von Gesichtsmasken bekannt, die durch Dotierung mit geeigneten Wirkstoffen als Drug-Delivery-Systeme verwendet werden können. Damit wird auch hier lediglich Collagen als Trägermaterial ohne konkrete Wirkung in einer der erfindungsgemäßen Indikationen offenbart. Die darin beschriebenen Collagenmaterialien werden auch unter der Bezeichnung Matricol® (MATRICOL®) zur Verwendung in der kosmetischen Behandlung vertrieben. Die vorteilhafte Wirkung von MATRICOL® beruht dabei auf der Entspannung irritierter Haut, Hautberuhigung, einem schnellen Abklingen von Rötungen sowie einer Unterstützung der Regenerationsfähigkeit der Haut. Derartige Matricol® Matrices zeichnen sich üblicherweise durch einen Collagenanteil von > 90 Gew.-% aus.

Die WO 09/1 15216 A1 beschreibt die Verwendung von Baobab-Extrakt zur Behandlung von entzündlichen oder allergischen Hauterkrankungen, sowie trockener und geschädigter Haut z.B. aufgrund von Neurodermitis, Pruritus, Psoriasis u.ä., wobei in einer Ausführungsform die erfindungsgemäßen Wirkstoff-Extrakte mittels schichtförmiger, gefriergetrockneter Collagenmatrices appliziert werden können. Auch hier werden die Collagenmatrices jedoch ausschließlich als Trägermaterialien ohne konkreten Hinweis auf eine eigene positive Wirkung in der Behandlung der beanspruchten Indikationen offenbart.

Gegenstand der DE 102004002990 A1 sind Drug-Delivery-Pads aus nanostrukturierten Fasernetzwerken, insbesondere aus bakteriell synthetisierter Cellulose, die einen hohen Kühlungseffekt bewirken und die außerdem zur Applikation von Wirkstoffen geeignet sind, wobei in der Gruppe möglicher Wirkstoffe u.a. auch lösliches Collagen aufgeführt ist. Darüber hinaus wird die Verwendung der erfindungsgemäßen Pads zur topischen Behandlung von Verletzungen, schmerzhaften Gewebeschädigungen sowie von Neurodermitis oder Ekzemen beschrieben, wobei die positiven Effekte in diesen Indikationen jedoch auf den besonderen Kühlungseffekt der erfindungsgemäßen Matrices zurückgeführt wird. Ein konkreter Anhaltspunkt, dass auch der beispielhaft aufgeführte zusätzliche Wirkstoff "lösliches Collagen" einen positiven Effekt in der entsprechenden Indikation entfaltet, ergibt sich hieraus ebenfalls nicht.

### AUFGABENSTELLUNG:

Die Aufgabe der vorliegenden Erfindung bestand darin, für die Behandlung von entzündlichen und degenerativen Hauterkrankungen sowie der damit verbundenen Hautschädigungen wie insbesondere Psoriasis, Dermatitis, Neurodermitis, Rosacea, Urticaria (Nesselsucht), Pruritus (Juckreiz), Hautekzemen und aktinischer Keratose, einschließlich der jeweils damit einhergehenden Symptome verbesserte Behandlungsmittel zur Verfügung zu stellen. Die im Rahmen der vorliegenden Erfindung überraschend gefundene vorteilhafte Wirkung des tierischen Collagens, insbesondere in Form gefriergetrockneter Auflagen, Masken oder Pads in den genannten medizinischen Indikationen war dabei aus dem Stand der Technik weder bekannt noch naheliegend.

### BESCHREIBUNG DER ERFINDUNG:

Entzündliche Hauterkrankungen im Sinne der Erfindung schließen beispielsweise ein systemische entzündliche Hauterkrankungen, Cyclooxygenase-abhängige entzündliche Hauterkrankungen und lokal induzierte entzündliche Hauterkrankungen wie solche, die beispielsweise in Folge einer lokalen Behandlung von aktinischen Keratosen mit Immunstimulierenden Substanzen wie zum Beispiel Imiquimod auftreten oder entzündliche und allergische Hauterkrankungen, z.B. allergische Hautreaktionen, als Folge von Unverträglichkeitsreaktionen oder Nebenwirkungen aufgrund medizinischer Behandlungen, einschließlich der jeweils damit einhergehenden Symptome und Begleiterscheinungen. Dabei sind entzündliche Hauterkrankungen im wesentlichen durch folgende direkte Entzündungszeichen oder Symptome am Ort der Entzündung gekennzeichnet: Rötung, Überwärmung, Schwellung, Schmerz und eingeschränkte Funktion, wobei diese Entzündungszeichen nicht immer direkt erkennbar oder auch nur teilweise nachweisbar sind. Häufige Begleiterscheinungen entzündlicher Hauterkrankungen sind außerdem Ödembildung, Blasen-, Pustel- oder Quaddelbildung, Ekzembildung sowie Pruritus (Juckreiz), wobei diese nicht zwangsläufig auftreten. Auch ist möglich, dass lediglich eine oder mehrere dieser Begleiterscheinungen auftreten. Daneben können je nach Schweregrad auch weitere unspezifische Entzündungszeichen in Form allgemeiner Reaktionen des Gesamtorganismus auftreten wie Fieber, allgemeines Krankheitsgefühl, Leukozytenanstieg oder - abfall, CRP-Anstieg, beschleunigte Blutkörperchensenkung oder Procalcitonin-Anstieg. Das erfindungsgemäß verwendete tierische Collagen wirkt dabei vorzugsweise positiv im Sinne von heilend oder lindernd auf die direkten Entzündungszeichen Rötung, Überwärmung (Hitze oder Brennen), Schwellung, Spannungsgefühl, Schmerz und eingeschränkte Funktion, sowie insbesondere auch auf die möglichen Begleiterscheinungen Ödembildung, Blasen-, Pustel- oder Quaddelbildung sowie insbesondere auch auf Pruritus (Juckreiz). Entzündliche Hauterkrankungen umfassen beispielsweise auch Akne.

Degenerative Hauterkrankungen schließen beispielsweise ein Psoriasis (Schuppenflechte), Dermatitis, (insbesondere auch atopische Dermatitis/ Neurodermitis), Rosacea, Urticaria (Nesselsucht), aktinische Keratose und Handekzem, einschließlich der jeweils damit einhergehenden Symptome.

Hautschädigungen im Sinne der vorliegenden Erfindung betreffen prinzipiell Funktionsstörungen oder Beeinträchtigungen der natürlichen physikalischen und mechanischen Schutz- und/oder Barriereeigenschaften der Haut und umfassen allgemein Hautschädigungen die durch physikalische (mechanische oder thermische Reize oder Strahlung), chemische (Säuren, Laugen, Toxine, Allergene) oder biologische Reize hervorgerufen werden, insbesondere auch trockene, rissige, schuppige oder schorfige Hautveränderungen sowie Hautveränderungen die gekennzeichnet sind durch Bildung von Papeln (Papula), Knoten (Nodus), Bläschen (Vesicula), Blasen (Bulla), Pusteln (Pustula), Quaddeln (Urtica), Schuppen (Squama), Krusten (Crusta), Fissuren (Rhagade), Erythembildungen z. B. auch in Verbindung mit Teleanglektasien (sichtbare erweiterte Kapillargefäße der Haut oder auch der oberen Dermis), was insbesondere bei Rosacea auftritt oder Erythema nach Kältestimulation wie durch Vereisung beispielsweise mittels flüssigem Stickstoff z.B. in der Behandlung von aktinischer Keratose, sowie ekzematöse Veränderungen der Haut, auch in Form von Blasenbildung, Quaddeln oder Pusteln, allergische Hautveränderungen sowie Dermatosen allgemein. Im übrigen können solche Hautschädigungen auch durch kosmetische oder medizinische Behandlungen mit mechanischer, chemischer, physikalischer oder thermischer Belastung, wie vorstehend beispielhaft genannt, hervorgerufen werden.

Klarstellend sei angemerkt, dass die Zuordnung der beschriebenen Störungsbilder, Symptome oder Begleiterscheinungen zu einzelnen der beschriebenen erfindungsgemäß umfassten Hauterkrankungen keine ausschließliche Zuordnung darstellt, Vielmehr können viele der genannten Symptome oder Störungsbilder im Zusammenhang mit nahezu allen der genannten Krankheitsbilder auftreten. So sind beispielsweise Erythem (Rötung), Überwärmung, Pruritus (Juckreiz), ekzematöse Veränderungen, oder Schuppung, die in unterschiedlich starken Ausprägungsgraden auftreten können, Leitsymptome oder Begleiterscheinungen von z.B. Rosacea, Psoriasis, Dermatitis, Neurodermitis, Dermatosen, Ekzemen etc.

Dabei wurden bei der erfindungsgemäßen Verwendung in den beschriebenen Indikationen bzw, bei den beschriebenen Symptomen insbesondere die folgenden positiven Effekte beobachtet:
- Beschleunigtes Abklingen der Symptome und damit einhergehend schmerzlindernde Wirkung
- juckreizlindernde bis -stillende Wirkung (Hautberuhigung)
- anhaltende Juckreizlinderung
- Langanhaltender Kühlungseffekt / Reduzierung von Hitze oder Brennen
- Abschwellende Wirkung
- Reduzierung des Spannungsgefühls
- Abklingen der Rötung
- Verbesserte Hautbefeuchtung
- Abnahme von Schuppung oder Trockenheitsrissen
- Erhöhte Haut-Elastizität (Hautoberfläche wird elastischer, spannungsärmer)
- Verbesserte Funktionsfähigkeit der betroffenen Körperteile (z.B. Verbesserung der Beweglichkeit von betroffenen Gelenkregionen wie Fingern, Ellbogen, Knie etc.)
- Angenehmes Hautgefühl während und nach der Applikation
- Verbessertes Hautgefühl nach der Applikation
- Hohe Verträglichkeit / Biokompatibilität

Insbesondere in einer Akutbehandlung von Pruritus (Juckreiz), meist als Symptom oder Begleiterscheinung einer der vorgenannten Erkrankungen oder Störungsbilder, durch die erfindungsgemäße Verwendung, vorzugsweise schichtförmiger Collagenmaterialien, zeigen sich die folgenden positiven Effekte und Vorteile gegenüber herkömmlichen Behandlungsmethoden, die üblicherweise in Form von Creme-Behandlungen erfolgen:
Primäre Effekte:
   - Unmittelbare Linderung bis Stillung des Juckreizes
   - Hautberuhigung
   - Erleichterung der akuten Beschwerden
   - Abnahme der meistens mit dem Pruritus einhergehenden Rötung
   - Hautpflege (ggf, auch durch weitere in dem Collagenmaterial enthaltene Hautpflege-Substanzen)
Sekundäre Effekte:
   - Kratzimpuls wird für die Einwirkzeit durch die physische Barriere durch Auflegen einer Schutzschicht auf die betroffenen Hautstellen in Form der schichtförmigen Auflage gehemmt, dadurch wird die akute Hautirritation vermindert und eine weitere mechanische Hautschädigung (durch Kratzen) verhindert. Im Gegensatz dazu verstärken Cremes in der Regel den Kratzimpuls durch das manuelle Auftragen
   - Die bedeckte Haut bekommt Zeit, sich zu "entspannen"
Tertiäre Effekte:
   - Juckreiz kann als "unsichtbares" Krankheits-Symptom angesehen werden, durch das Auflegen eines deutlich sichtbaren Produkts wird dem Betroffenen eine Krankheitsanerkennung zuteil (psychologischer Effekt)

Erfindungsgemäß bevorzugt ist die Verwendung von tierischem Collagen in der Behandlung von Psoriasis, Dermatitis, Neurodermitis, atopischer Dermatitis, Rosacea, Urticaria (Nesselsucht), Pruritus (Juckreiz) und Hautekzemen bzw. ekzematösen Veränderungen, Erythema und aktinischer Keratose, insbesondere von anhaltenden entzündlichen Läsionen nach Abschluß einer lokalen Behandlung aktinischer Keratose mit Immunstimulanzien, sowie jeweils der damit einhergehenden Symptome.

Besonders bevorzugt ist die Verwendung von Collagen in der Behandlung von Psoriasis, Dermatitis, Neurodermitis, atopischer Dermatitis, Rosacea, Urticaria (Nesselsucht), Pruritus (Juckreiz) sowie anhaltenden entzündlichen Läsionen nach Abschluß einer lokalen Behandlung aktinischer Keratose mit Immunstimulanzien, sowie jeweils der damit einhergehenden Symptome

Dabei ist die Verwendung von tierischem Collagen in der Behandlung von Neurodermitis, atopischer Dermatitis, Rosacea, Urticaria (Nesselsucht), Pruritus (Juckreiz), sowie anhaltenden entzündlichen Läsionen nach Abschluß einer lokalen Behandlung aktinischer Keratose mit Immunstimulanzien, sowie jeweils der damit einhergehenden Symptome ganz besonders bevorzugt.

Am meisten bevorzugt ist die erfindungsgemäße Verwendung in der Behandlung von Pruritus (Juckreiz).

Die überraschende vorteilhafte Wirkung in der Behandlung der vorstehend genannten medizinischen Indikationen bzw. Symptome wurde insbesondere für tierisches Collagen beobachtet, welches aus der Gruppe der tierischen Collagene ausgewählt ist. Dieses wird vorzugsweise aus Collagenquellen bovinen, equinen und porcinen Ursprungs gewonnen, wobei bovines Collagen ganz besonders bevorzugt ist. Das Collagen kann nach üblichen Verfahren aus den üblichen Quellen wie Häuten oder Sehnen gewonnen werden und vorzugsweise nach den aus dem Stand der Technik und z. B. aus der DE 4028622 A1 oder aus der DE 10350654 A1 bekannten Verfahren aufgearbeitet und hergestellt werden.

Die erfindungsgemäß bevorzugten Collagene und Collagenmaterialien zeichnen sich insbesondere durch herausragende Hydratationseigenschaften und eine gute Flüssigkeitsaufnahmekapazität bzw. Saugfähigkeit aus. Aufgrund der strukturellen Ähnlichkeit mit der menschlichen Haut und menschlichem Gewebe werden bevorzugt Collagenarten ausgewählt, die in Haut und Gewebe auftreten, insbesondere Collagen des Typs I, III und V. Dadurch bedingt sich die besonders gute Verträglichkeit und Biokompatibilität solcher erfindungsgemäß verwendeter Collagene.

Eine weitere Ausführungsform betrifft solche tierischen Collagene, die folgenden Fraktionen umfassen:
a) faserförmig unlösliches Collagen
b) nativ-lösliches (säurelösliches) Collagen und
c) Collagen-Peptide.

Unter faserförmig unlöslichem Collagen gemäß Fraktion a) versteht man die in saurer Lösung von pH <4 unlösliche, durch Zentrifugation bei 16.000 g präzipitierbare Fraktion einer Collagensuspension, die Fasern enthält, welche im Lichtmikroskop sichtbar sind (Faserdicke ab 0,2 µm).

Nativ-lösliches bzw. säurelösliches Collagen bezeichnet den Anteil an Collagen, der in saurer Lösung bei pH <4 eine klare Lösung bildet, welche keine unter dem Lichtmikroskop erkennbaren Faserstrukturen beinhaltet. Das nativ-lösliche bzw. säurelösliche Collagen kann durch Fraktionierung, beispielsweise mittels SEC (Size-Exclusion-Chromatographie), in höhermolekulare, vollständige Collagenmoleküle mit einem Molekulargewicht >250kDa, entsprechend Fraktion b), und niedermolekulare Collagenpeptide mit einem Molekulargewicht <250kDa getrennt werden.

Damit bezeichnet vorstehend genannte Fraktion b) nativ-säurelösliche höhermolekulare, vollständige Collagenmoleküle mit einem Molekulargewicht >250kDa.

Collagen-Peptide gemäß Fraktion c) umfassen dementsprechend den niedermolekularen Anteil der säure-löslichen Fraktion, welcher mit einem Molekulargewicht von <250kDa keinem vollständigen Collagenmolekül zugeordnet werden kann.

Das Vorliegen von löslichen vollständigen Collagenmolekülen sowie der niedermolekularen Peptid-Bestandteile ist durch Analyse der löslichen Bestandteile mittels bekannten proteinchemischen Methoden, beispielsweise SDS-PAGE, qualitativ nachweisbar.

Bei Verwendung der erfindungsgemäßen tierischen Collagene, insbesondere wenn diese in Form rehydratisierbarer gefriergetrockneter schichtförmiger Collagenmatrices vorliegen, werden bei der Anwendung lösliche Komponenten aus den Fraktionen b) und c) freigesetzt und können unmittelbar auf dem Applikationsort ihre Wirkung entfalten. Dies ist insofern vorteilhaft, als solche löslichen Collagenfraktionen und niedermolekularen Peptidbestandteile eine filmbildende Wirkung aufweisen und darüber das Wasserhaltevermögen der Haut verbessern und entsprechend den transepidermalen Wasserverlust verringern.

Desweiteren ist es auch möglich, tierisches Collagen zu verwenden, welches einer Vernetzungsreaktion unterworfen wurde. In diesem Fall ist eine thermische Vernetzung, die so genannte Dehydrothermalvernetzung, bevorzugt. Ferner ist die Vernetzung mit chemischen Vernetzern möglich. Hierzu zählen insbesondere Aldehyde, wie Glutaraldehyd; Carbodiimide, wie EDC; Isocyanate; Epoxide oder Imidazole, wobei bi- und polyfunktionelle Epoxide aus der Gruppe der chemischen Vernetzer besonders bevorzugt sind.

Für die erfindungsgemäße Verwendung, die in der Regel topisch bzw. äußerlich erfolgt, ist besonders die Verwendung von Collagen in Form fester, trockener oder vorbefeuchteter, absorbierender bzw. hydratisierbarer Zubereitungen, insbesondere in Form von schichtförmigen, flächigen Auflagen geeignet. Damit ist erfindungsgemäß bevorzugt, das Collagen in Form von Masken, Sheets, Matrices, Auflagen, Pads, Lagen oder ähnlichen flächigen Formen in der erfindungsgemäßen Indikation zu verwenden, da sich solche Ausführungsformen besonders für die äußerliche und flächige Behandlung auch größerer betroffener Regionen der Haut eignen. Dabei wird weiterhin bevorzugt Collagen in Form poröser, schwammartiger, rehydratisierbarer, hydrophiler Ausführungsformen verwendet.

Besonders bevorzugt wird gefriergetrocknetes Collagen verwendet, ganz besonders bevorzugt sind gefriergetrocknete schichtförmige Collagenmatrices oder -auflagen. Die Verwendung gefriergetrockneter Collagenmatrices ist insofern bevorzugt, als es damit möglich wird, Collagenzubereitungen zur Verfügung zu stellen, die im Wesentlichen frei von Konservierungsstoffen und/oder Parfümstoffen sind. Konservierungs- und Parfümstoffe sind bekannt für ihr hautreizendes und irritatives Potential und können insbesondere bei vorgeschädigter Haut aufgrund der erfindungsgemäßen Hauterkrankungen und Störungsbilder den Heilungserfolg negativ beeinflussen. Entsprechend sind erfindungsgemäß besonders bevorzugt solche Ausführungsformen, die im Wesentlichen frei von Konservierungsstoffen und/oder Parfümstoffen sind.

Die erfindungsgemäß verwendeten Collagenmaterialien oder - zubereitungen bestehen aus mindestens 50 Gew.-% Collagen, bevorzugt aus mindestens 75 Gew.-% Collagen, bevorzugter aus mindestens 85 Gew.-% Collagen, ganz besonders bevorzugt aus > 90 Gew.-% Collagen.

Solche festen, schichtförmigen, rehydratisierbaren Collagenmatrices, vorzugsweise solche in Form gefriergetrockneter poröser, schwammförmiger Collagenmatrices zeichnen sich durch eine hohe Flüssigkeitsaufnahme- und -haltekapazität auf. Diese ist einerseits auf die hohe Flüssigkeitseinlagerungskapazität in die poröse Schwammstruktur solcher Matrices zurückzuführen sowie außerdem auf die Fähigkeit des Collagens große Mengen Wasser in Form von Hydrathüllen an den Collagenfasern und -fibrillen zu binden. Dadurch wird eine langanhaltende Hautbefeuchtung und ein hoher Feuchtigkeitseintrag in das behandelte Hautgewebe unterstützt, was sich vorteilhaft auf die Elastizität, Hautbefeuchtung und Hautglättung auswirkt. Daneben kann durch diesen Effekt solcher poröser, schwammartiger schichtförmiger Collagenmatrices eine langanhaltende, gleichmäßige Verdunstungswirkung auf dem Applikationsort und dadurch eine dauerhafte, gleichmäßige und anhaltende Kühlung bewirkt werden, was insbesondere bei aufgrund von Entzündungsreaktionen stark durchbluteten und überwärmten Hautarealen, den dadurch ausgelösten Hitzeempfindungen und Spannungsgefühlen sowie bei Pruritus (Juckreiz) und mit Juckreiz einhergehenden Hauterkrankungen vorteilhaft ist.

Schichtförmige Collagenmaterialien können im Grunde in jeder gewünschten geometrischen Form und Größe bereitgestellt werden oder individuell auf die Form des zu behandelnden Hautareals zugeschnitten werden. Für eine optimale Applizierbarkeit sind insbesondere schichtförmige Collagenmaterialien geeignet, die eine Schichtdicke (kürzeste Seitenlänge) von maximal 8 mm, bevorzugt bis 5 mm, bevorzugter bis 3 mm aufweisen.

Die erfindungsgemäß verwendeten Collagene, Collagenmatrices und Collagenzubereitungen, insbesondere solche in Form gefriergetrockneter schichtförmiger Matrices weisen einen pH-Wert < 7 auf. Bevorzugt ist der pH-Wert < 6, besonders bevorzugt < 5. Ein solcher saurer pH-Wert ist einerseits durch die oben genannten Herstellverfahren bedingt und darüber hinaus auch erwünscht, da solche pH-Werte dem natürlichen pH-Wert der Haut entsprechen bzw. nahe kommen. Die erfindungsgemäß bevorzugten niedrigen pH-Werte wirken insbesondere günstig auf die Verbesserung und Regeneration des natürlichen Säureschutzmantels der Haut. Es ist bekannt, dass der pH-Wert der Haut bei Hauterkrankungen wie beispielsweise Neurodermitis stark erhöht ist und in der Regel bis pH 8 ansteigen kann, was eine Schädigung des Säureschutzmantels anzeigt. Dadurch sind die erfindungsgemäßen Collagene-, Collagenmatrices und Collagenzubereitungen mit den bevorzugten sauren pH-Werten insbesondere auch in der Behandlung von Hauterkrankungen geeignet, die mit erhöhtem pH-Wert bzw. Schädigung des Säureschutzmantels einhergehen, wie z.B. Neurodermitis.

Die Verwendung der erfindungsgemäßen schichtförmigen Collagenmatrices ist darüber hinaus insofern besonders bevorzugt, da dadurch das Collagen quasi hochkonzentriert angewendet wird und die Kombination seiner vorteilhaften Eigenschaften optimal in der erfindungsgemäßen Verwendung zusammenwirken kann, was die überraschenden Effekte erst ermöglicht. Solche schichtförmigen Collagenmatrices entfalten insbesondere einen Synergismus durch die besonderen Hydratationseigenschaften zur Verbesserung der Hautbefeuchtung durch hohen Feuchtigkeitseintrag in die Haut und Verbesserung der Elastizität sowie intensive Kühlung, den bevorzugt niedrigen pH-Wert zur Regeneration des natürlichen Säureschutzmantels der Haut und der Freisetzung löslicher Collagen- und Peptidbestandteile zur Steigerung des Wasserhaltevermögens und damit Verminderung des transepidermalen Wasserverlustes sowie der Einschleusung hautähnlicher Collagen- und Peptidbestandteile zur Verbesserung der Elastizität und Regeneration der angegriffenen Haut. Eine Kombination dieser vorteilhaften Eigenschaften kann mit den zur Behandlung der erfindungsgemäßen Hauterkrankungen aus dem Stand der Technik bekannten Materialien und Zubereitungen nicht erreicht werden und die damit erzielbaren deutlichen Verbesserungen in der Wirkung waren so auch nicht vorhersehbar und damit überraschend.

Die erfindungsgemäß verwendeten tierischen Collagene können erfindungsgemäß in Form von Collagenzubereitungen vorliegen, bevorzugt in Form fester, trockener Collagenzubereitungen, die außerdem mindestens einen Wirk- und/oder Hilfsstoff enthalten. Solche Collagenzubereitungen sind beispielsweise unter der Bezeichnung Matricol®, wie vorstehend erwähnt, als kosmetische Behandlungsmittel auf dem Markt erhältlich.

Wirkstoffe schließen insbesondere kosmetische oder therapeutische bzw. pharmazeutische, für die äußere Anwendung geeignete Wirkstoffe ein. Kosmetische Wirkstoffe im Sinne der Erfindung sind im wesentlichen Mittel im Sinne des Lebensmittel-, Bedarfsgegenstände- und Futtermittelgesetzbuches (LFGB), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen.

Beispiele kosmetisch gegebenenfalls auch zum Beispiel dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Dexpanthenol (Panthenol, Pantothenol), Glycerin oder Harnstoff, Sonnenschutzmittel, Keratolytika, Radikalfänger für freie Radikale, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat, L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern, Alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, oder dekorative Stoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc. sowie abrasive Mittel.
Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoslde), Crataegus-Extrakt (z,B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl bzw. Johanniskrautextrakt, Nachtkerzenöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstlmulantien: Echinacea purpurea (z.B.alkoholische Auszüge, Frischpflanzensaft, Preßsaft), Eleutherokokkus senticosus; Alkaloide: Koffein, Teein, Schwarztee bzw. Schwarztee-Extrakt, Theobromin, Capsaicin, Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginseno-side), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B. Urtinktur), Mistel (z.B. wässrig-ethanol. Auszug), Phytosterole (z.B. beta-Sitosterin), Wollblumen (z.B. wäßrig-alkohol, Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte, Cardiospermum Urtinktur, Dulcamara Extrakt, sowie Gerbstoffe wie Tannin.

Im Unterschied zu den vorstehend beschriebenen im wesentlichen in der Kosmetik verwendeten Wirkstoffen handelt es sich bei den therapeutischen Wirkstoffen (Arzneimitteln) um solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Erfindungsgemäß sind insbesondere solche Mittel bzw. Wirkstoffe geeignet, die für die äußere dermale oder transdermale Anwendung, insbesondere im Bereich der Hautbehandlung und -heilung bestimmt sind.

Bei Wirkstoffen für eine dermale oder transdermale Anwendung handelt es sich insbesondere um hautaktive aber auch um transdermale Wirkstoffe. Sie schließen beispielsweise ein: Mittel zur Behandlung von Brandverletzungen, Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (Entzündungshemmer) (NSAR), z. B. Weihrauch bzw. Weihrauchextrakt, Indometacin, Diclofenac, Naproxen, Ketoprofen, Ibuprofen, Flurbiprofen, Salicylsäure und -derivate wie Acetylsalicylsäure, Oxicame; Steroidhormone, z. B. Corticoide und Glucocorticoide wie Hydrocortison, Cortisol, Cortisonacetat, Cloprednol, Prednison, Prednisolon, Deflazacort, Fluocorolon, Triamcinolon, Betamethason, Betamethasonvalerat, Mometasonfuroat, Dexamethason, Methylprednisolon, Ethinylestradiol, Medroergotamin, Dihydroergotoxin; Gichtmittel, z. B. Benzbromaron, Allopurinol; Dermatika Externa; Antihistaminika wie Brompheniramin, Bamipin; Antibiotika wie Erythromycin, Clindamycin, Tetracyclin, einschließlich antibakterielle Mittel wie z.B. kolloidales Silber und Silbersalze wie Silberchlorid, Silbernitrat, Silberjodid oder weitere aus dem Stand der Technik bekannte Silber-haltige Wundbehandlungsstoffe; Antimykotika; Peptidarzneistoffe; antivirale Wirkstoffe; entzündungshemmende Wirkstoffe; juckreizstillende Wirkstoffe wie z. B. anästhesierende Wirkstoffe, Antihistaminika, Benzocain, Polidocanol oder Corticoide und Glucocorticoide; Aknemittel; antiparasitäre Wirkstoffe; äußerlich anwendbare Hormone; Venentherapeutika; Immunsuppresiva wie Calcineurin-Inhibitoren wie Tacrolimus und Pimecrolimus, Mineralstoffe und Spurenelemente, wie z.B. anorganische oder organische Selenverbindungen, Zink und Zinksalze etc. alle für die dermale oder transdermale Anwendung.

Klarstellend sei angemerkt, dass die Einordnung der Wirkstoffe in die Gruppe der kosmetischen oder therapeutischen Wirkstoffe im Rahmen der vorliegenden Erfindung keine ausschließliche Zuordnung darstellt. Insbesondere schließt die hier vorgenommene Einordnung nicht aus, dass die entsprechenden Wirkstoffe sowohl als kosmetische sowie auch als therapeutische Wirkstoffe Anwendung finden.

Bevorzugte Wirkstoffe für die dermale und transdermale Anwendung sind ausgewählt aus der Gruppe die enthält: Mittel zur Behandlung von Hautkrankheiten wie der Neurodermitis, der atopischen Dermatitis, Psoriasis, Rosacea etc., entzündungshemmende Wirkstoffe, juckreizstillende Wirkstoffe, Gerbstoffe, topische Analgetika, Anästhetika und antibakterielle Wirkstoffe, Besonders bevorzugte Wirkstoffe sind ausgewählt aus Silber-haltigen Wirkstoffen wie insbesondere Silbernitrat, Silberchlorid, Microsilber-Partikel, Tacrolimus, Pimecrolimus, Antihistaminika, Polidocanol, Weihrauch/Weihrauchextrakt, Capsaicin, Tannin, Johanniskrautöl/Johanniskrautextrakt, Nachtkerzenöl, Dexpanthenol sowie anorganischen oder organischen Selenverbindungen, Zink und Zinksalzen.

Ganz besonders bevorzugt ist mindestens ein Wirkstoff ausgewählt aus der Gruppe der hautähnlichen Lipide, umfassend beispielsweise Phospholipide, neutrale Lipide und Sphingolipide sowie Komponenten des natürlichen Feuchthaltefaktors der Haut, des sogenannten Natural Moisturizing Factors (NMF), umfassend beispielsweise Harnstoff (Urea), Aminosäuren und Carbonsäuren, Pyrrolidoncarbonsäure, Natrium, Kalium, Calcium, Magnesium, Laktat (Milchsäure), Citrat, Chlorid, Phosphat etc., Harnsäure und andere organische Säuren.

Die erfindungsgemäß verwendeten Collagene, insbesondere solche in Form von Collagenzubereitungen, können außerdem mindestens einen Hilfsstoff enthalten.

Hilfsstoffe schließen ein: pH-Einstellungsmittel, wie Pufferstoffe, anorganische und organische Säuren oder Basen; Fettsubstanzen, wie Mineralöle, wie Paraffinöle oder Vaselineöle, Siliconöle, Pflanzenöle wie Kokosöl, Mandelöl bzw. Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamomöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Squalan, Fettsäureester, Ester von Fettalkoholen wie Triglyceride, und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Hand-book, 1. Auflg., 1988, The Cosmetic, Toiletry and Fragrance Association, Inc., Washington, erwähnt, oberflächenaktive Mittel neben den oben erwähnten Waschtensiden, wie Dispergiermittel, Netzmittel, Emulgatoren etc.; Füllstoffe; Stabilisatoren; Cosolventien; pharmazeutisch und kosmetisch gebräuchliche oder sonstige Farbstoffe und Pigmente, insbesondere solche, die primär zur farblichen Gestaltung der Collagen-Zusammensetzung eingesetzt werden und nicht zur Applikation und farblichen Gestaltung am menschlichen Körper, wie solche Pigmente und Farbstoffe wie die unter der Gruppe der kosmetischen Wirkstoffe aufgeführten dekorativen Farbstoffe; Konservierungsmittel; Weichmacher; Schmiermittel bzw. Gleitmittel; etc. Aus oben genannten Gründen sind Hilfsstoffe aus der Gruppe der Konservierungsstoffe jedoch weniger bevorzugt.

Erfindungsgemäß bevorzugte Hilfsstoffe sind Fette und Öle. Dabei sind insbesondere kosmetische Öle wie vorstehend aufgezählt, insbesondere Triglyceride, besonders bevorzugt Capryl/Capronsäure-Triglyceride, Squalan oder Jojobaöl sowie Nachtkerzenöl.

Ganz besonders bevorzugt sind aus der Gruppe der Fette und Öle die unter der vorstehend definierten Gruppe der Wirkstoffe genannten hautähnlichen Lipide, die in die Untergruppen polare Lipide, neutrale Lipide und Sphingolipide eingeordnet werden können. In der Gruppe der polaren Lipide sind insbesondere Phospholipide und Cholesterylsulfat zu nennen, zu den bevorzugten neutralen Lipiden gehören Squalan, Squalen, Triglyceride, Wachsester, Sterole (z.B. Cholesterol) und sowohl gesättigte als ungesättigte freie Fettsäuren, bevorzugte Sphingolipide sind insbesondere die gesamte Gruppe der Ceramide.

Generell schließt die Einordnung der vorstehend erwähnten Stoffe in die Kategorie der Hilfsstoffe im Rahmen der vorliegenden Erfindung nicht aus, dass diese Hilfsstoffe auch gewisse kosmetische und/oder therapeutische Wirkungen entfalten können, was in besonderem Maß für die genannten bevorzugt eingesetzten kosmetischen Öle gilt. Insbesondere die in der Gruppe der Wirkstoffe genannten, bevorzugten hautähnlichen Lipide sind prinzipiell gleichermaßen durch die unter den Hilfstoffen genannten Gruppe der Fette und Öle erfasst.

Eine besondere Ausführungsform betrifft die erfindungsgemäße Verwendung von Collagen bzw. einer Collagenzubereitung im Sinne der vorliegenden Erfindung, welche im Wesentlichen frei von Hilfsstoffen ist, die aus der Gruppe der Konservierungsstoffe und Parfümstoffe sowie der Polyether, wie insbesondere aus der Gruppe der Polyethylenglykole (PEGs), Polypropylenglykole (PPGs) und Polyglykole (PGs), ausgewählt sind.

Weiterhin können die schichtförmigen Collagenmatrices und - zubereitungen auch laminiert sein bzw. in Form mehrschichtiger, miteinander verbundener Lagen ("sandwich-layer") vorliegen, Als Laminate können übliche, aus dem Stand der Technik bekannte Materialien wie z.B. Fasern, Vliese, Netze, Filme oder Folien aus geeigneten Materialien wie z.B. Rayon, Cellulose, Polyethylen (PE) oder Polyurethan (PU) oder anderen synthetischen oder semi-synthetischen Polymeren / Copolymeren verwendet werden, die mit herkömmlichen Methoden, z.B. durch Kleben, Heißlaminieren, Vernetzen etc. mit den Trägermaterialien im Sinne der vorliegenden Erfindung fest verbunden werden können. Eine derartige Laminierung ist besonders geeignet, um die mechanische Stabilität der erfindungsgemäßen schichtförmigen Collagenmaterialien zu erhöhen, sowie deren Handhabbarkeit bei der Applikation, besonders im befeuchteten Zustand zu verbessern. Eine bevorzugte Laminierung umfasst eine Laminierung mit einer selbstklebenden Schicht bwz. einem schichtförmigen Pflastermaterial, welches bevorzugt so auf den schichtförmigen Collagenmaterialien angebracht wird, dass die selbstklebende Laminatschicht randseitig ganz oder teilweise über das Collagenmaterial hinausragt, so dass die laminierten Collagenmaterialien ähnlich einer herkömmlichen Pflasteranordnung mittels der randseitig überstehenden selbstklebenden Laminierung auf den zu behandelnden Hautarealen leicht fixiert werden können. Bei solchen selbstklebenden laminierten Collagenmaterialien sind insbesondere solche selbstklebenden Laminierungsbeschichtungen bevorzugt, die über eine besonders hohe Hautverträglichkeit, gering Irritations- und Allergieneigung sowie eine leichte Ablösbarkeit verfügen, um die bereits vorgeschädigten oder irritierten Hautareale beim Ablösen der Klebeschicht nicht zusätzlich zu irritieren. Desweiteren sind besonders solche selbstklebenden Laminierungsbeschichtungen bevorzugt, die hydrophil und nicht-okklusiv oder maximal semi-okklusiv sind, um die Befeuchtung der laminierten Collagenmaterialien für die Anwendung zu ermöglichen. Okklusive Laminierungsbeschichtungen sind insofern nicht bevorzugt, da diese keine Verdunstung ermöglichen und damit nachteilig hinsichtlich des vorteilhaften Effekts der Verdunstung und damit langanhaltenden Kühlung der erfindungsgemäßen Collagenmaterialien sind. Desweiteren ist bei der Wahl solcher selbstklebender Laminierungsbeschichtungen darauf zu achten, dass die Klebeschicht nicht wasserlöslich ist, damit beim Befeuchten des Materials der Kleber nicht herausgelöst werden kann und damit die Klebe- bzw. Fixierungswirkung verloren geht.

Damit umfasst die vorliegende Erfindung insbesondere auch solche schichtförmigen Collagenmaterialien, welche eine zusätzliche Schicht aus Fasern, Vliesen, Netzen, Filmen oder Folien oder eine selbstklebende Schicht aufweisen wobei diese zusätzliche Schicht das Collagenmaterial teilweise oder vollständig bedeckt und so auf dem schichtförmigen Collagenmaterial aufgebracht ist, dass es mit diesem randseitig abschließt oder randseitig vollständig oder partiell über das Collagenmaterial hinausragt,

Bei der erfindungsgemäßen Verwendung von Collagen bzw. der Collagenzubereitungen in der bevorzugten Applikationsform schichtförmiger, trockener Auflagen, Pads oder Masken umfasst die Behandlung im wesentlichen die folgenden Schritte:
a) Bereitstellen einer schichtförmigen, trockenen Applikationsform aus Collagen oder einer Collagenzubereitung
b1) Befeuchten des trockenen schichtförmigen Collagenmaterials mit einer wässrigen Lösung und Aufbringen des befeuchteten Collagenmaterials auf die zu behandelnden Hautareale oder
b2) Aufbringen des trockenen schichtförmigen Collagenmaterials auf die zu behandelnden Hautareale und Befeuchten des trockenen schichtförmigen Collagenmaterials auf den zu behandelnden Hautarealen,

Die befeuchteten Collagenmaterialien können dabei im Prinzip bis zum vollständigen Trocknen auf dem zu behandelnden Hautareal verbleiben. Bevorzugt werden die befeuchteten, schichtförmigen Collagenmaterialien jedoch nicht länger als 60 Minuten, bevorzugter bis zu 45 Minuten auf der Haut belassen. Die befeuchteten schichtförmigen Collagenmaterialien werden bevorzugt mindestens 5 Minuten, bevorzugter mindestens 10 Minuten, besonders bevorzugt mindestens 20 Minuten auf dem zu behandelnden Hautareal belassen. Danach werden die in der Regel noch feuchten Collagenmaterialien abgenommen und ggf, kann anschließend eine weitere herkömmliche Pflegebehandlung der behandelten Hautareale mit üblichen Pflegeprodukten durchgeführt werden.

Bei der erfindungsgemäßen Verwendung solcher schichtförmigen befeuchteten Collagenmaterialien erfolgt die Befeuchtung bzw. Rehydratisierung der schichtförmigen - bevorzugt gefriergetrockneten - Collagenmaterialien, wie beispielsweise vorstehend in Schritt b1) oder b2) beschrieben, mit einer wässrigen Lösung die ausgewählt ist aus der Gruppe umfassend Wasser sowie ggf. entmineralisiertes Wasser oder sogenanntes Thermalwasser, physiologische Lösungen (z.B. physiologische Kochsalzlösungen) sowie wässrige Lösungen, die mindestens einen Wirk- und/oder Hilfsstoff enthalten. Die zur Befeuchtung bzw. Rehydratisierung verwendeten wässrigen Lösungen werden auch als Aktivatorlösungen bezeichnet.

Bei solchen Aktivatorlösungen kann es sich beispielsweise um Lösungen von leicht-flüchtigen Wirk- und/oder Hilfsstoffen handeln, die aufgrund des Herstellverfahrens z.B. durch die Gefriertrocknung nicht in ein gefriergetrocknetes Material eingebracht werden sollen bzw. können, wie z.B. gewisse Anteile ätherischer Öle, Parfums etc. Auch können solche Wirk- und/oder Hilfsstoffe enthalten sein, die eine zusätzliche befeuchtende Wirkung erzielen und die aufgrund dieser befeuchtenden Wirkung oder aufgrund von Hygroskopie-Neigungen nicht oder nur in geringen Mengen in die erfindungsgemäß bevorzugten gefriergetrockneten Zubereitungen eingearbeitet werden können, da dadurch entweder die Stabilität des gefriergetrockneten Collagenmaterials selbst oder die Stabilität eventuell enthaltener feuchtigkeitslabiler Wirkstoffe nicht mehr aufrechterhalten werden kann.

Grundsätzlich können in den Aktivatorlösungen einer oder mehrere der vorstehend genannten Wirk- und/oder Hilfsstoffe enthalten sein. Insbesondere sind Wirkstofflösungen besonders geeignet, die einen oder mehrere der vorstehend genannten bevorzugten Wirk- oder Hilfsstoffe zur therapeutischen Anwendung in den erfindungsgemäßen Indikationen enthalten.

In einer besonders bevorzugten Ausführungsform wird eine wässrige Aktivatorlösung verwendet, die im Wesentlichen frei von Emulgatoren, Konservierungsstoffen, Parfümstoffen und/oder Hilfsstoffen aus der Gruppe der Polyether, Polyethylenglykole (PEGs), Polypropylenglykole (PPGs) und Polyglykole (PGs) ist.

In einer weiteren bevorzugten Ausführungsform der Erfindung liegt das erfindungsgemäße Collagenmaterial mit der Aktivatorlösung in einer zusammengehörenden, räumlichen Anordnung (Anwendungspaket, Set, Kit-of-Parts etc.) vor. Derartige Kit-of-parts Kombinationen umfassen bevorzugt mindestens eines der erfindungsgemäßen Collagenmaterialien, bevorzugt solche in Form schichtförmiger Auflagen, Pads oder Masken, sowie mindestens eine wässrige Lösung, die einen oder mehrere Wirkstoffe und/oder mindestens einen oder mehrere Hilfsstoffe enthält (Aktivatorlösung),

Die Ausgestaltung solcher Kit-of-parts Kombinationen aus erfindungsgemäßem Collagenmaterial einerseits und Aktivatorlösung andererseits kann dabei vorsehen, dass die beiden Bestandteile separat aus der Kit-of-parts Anordnung entnommen werden und außerhalb davon für die weitere Verwendung zusammengeführt werden. Es ist jedoch auch denkbar, dass eine Zusammenführung der Komponenten innerhalb der Kit-of-parts-Verpackung, z.B. in dafür vorgesehenen Kammern, selbst erfolgt und die rehydratisierte Zusammensetzung sodann aus dieser direkt der weiteren äußerlichen oder transdermalen Verwendung zugeführt wird. Dies kann bevorzugt direkt durch den Endverbraucher durchgeführt werden.

Erfindungsgemäß erfolgt die Verwendung der Collagenmaterialien bevorzugt äußerlich bzw. dermal oder topisch.

Klarstellend sei angemerkt, dass im Rahmen der vorliegenden Erfindung die verschiedenen Ausführungsformen des Collagens auch zusammenfassend mit dem Begriff "Collagenmaterial" bezeichnet werden.

Die vorliegende Erfindung wird durch die folgenden Beispiele weiter verdeutlicht. Die Beispiele stellen lediglich Exemplifizierungen dar, und der Fachmann ist in der Lage die spezifischen Beispiele auf weitere beanspruchte Ausführungsformen auszudehnen.

### BEISPIELE

### Beispiel 1

Verwendung von gefriergetrockneten schichtförmigen Collagenmatrices in der Behandlung von Neurodermitis

Die Behandlung einer seit Geburt bestehenden Neurodermitis-Erkrankung eines männlichen Patienten (Alter: 39 Jahre) erfolgte mit verschiedenen Ausführungsformen schichtförmiger gefriergetrockneter Collagenmaterialien gemäß der vorliegenden Erfindung.

Eingesetzt wurden:
a) Schichtförmige gefriergetrocknete Collagenmatrices aus reinem (bovinem) Collagen (100 Gew.-% Collagen) (ohne weitere Wirk- oder Hilfsstoffe), die nach dem erfindungsgemäß als bevorzugt beschriebenen Verfahren erhältlich sind
b) Verschiedene handelsübliche schichtförmige gefriergetrocknete Collagenzubereitungen der Marke Matricol®, jeweils enthaltend > 90 Gew.-% Collagen sowie außerdem diverse übliche Wirk- und/oder Hilfsstoffe wie im Rahmen der vorliegenden Erfindung definiert, beispielsweise Dexpanthenol, grüner Tee-Extrakt, Kaviar-Extrakt, Squalan, kosmetische Fette und Öle.

Vor der Anwendung des Collagenmaterials war die Haut jeweils stark gerötet und gespannt sowie stark juckend

Für die Behandlung wurden die schichtförmigen Collagenmaterialien auf die Größe der betroffenen Hautareale zugeschnitten, auf die zu behandelnde Haut trocken aufgelegt und durch Besprühen mit Wasser (Leitungswasser) bis zur Sättigung befeuchtet. Die rehydratisierten Collagenauflagen wurden für einen Zeitraum von ca. 30 min auf den betroffenen Hautstellen belassen.

Während dieser Behandlung zeigte sich ein deutliches Abklingen der Rötung sowie eine deutliche Linderung des Juckreizes. Die behandelte Haut war entspannt und damit die Funktionsfähigkeit deutlich verbessert. Diese positiven Effekte der Behandlung waren anhaltend über mindestens 6h, bei Wiederholung der Behandlung auch über 12h.

Die beobachteten Effekte zeigten sich gleichermaßen sowohl bei reinen Collagenmaterialien mit 100 Gew.-% Collagen gemäß a) sowie den diversen Wirk- /Hilfsstoffhaltigen Matricol®-Varianten mit einem Collagengehalt > 90 Gew.-% gemäß b).

### Beispiel 2

### Verwendung von gefriergetrockneten schichtförmigen Collagenmatrices in der Behandlung von Rosacea

Die Behandlung erfolgte im Rahmen einer klinischen Untersuchung (Studie/Anwendungsbeobachtung) mittels handelsüblichen schichtförmigen gefriergetrockneten Collagenzubereitungen der Marke Matricol® mit einem Collagengehalt > 90 Gew.-%.

Im Rahmen der Untersuchung führten 10 Patienten im Alter von 24 bis 79 Jahren mit leichter Ausprägung (Rosazeadiathese und R. erythematosateleangiectatica) bzw. schwerer Ausprägung (R. papulopustulosa) der Rosacea-Erkrankung jeweils bis zu 6 Einzelanwendungsbeobachtungen durch. Insgesamt wurden von den 10 Patienten 41 Einzelanwendungen durchgeführt, wobei in 4 Anwendungsfällen Patienten das Ergebnis nach 24 Stunden nicht dokumentierten.

Dazu beurteilten die Patienten die folgenden Parameter ca. 15 Minuten nach erfolgter Matricol-Anwendung sowie 24 Stunden nach der Behandlung jeweils im Vergleich zum Zeitpunkt vor der Anwendung am jeweiligen Anwendungstag. Außerdem erfolgte ca. 15 Minuten nach der Matricol-Anwendung auch eine entsprechende Beurteilung der Parameter durch den Prüfarzt.

### Beurteilungsparameter:

Abnahme der Rötung
Hautberuhigung
Angenehmes Hautgefühl
Normalisierung der Durchblutung
Ausprägung der Teleangiektasie (Arztbeurteilung)
Reduzierung von Empfindungen wie Brennen, Jucken, Kribbeln, Taubheit
(Patientenbeurteilung nach 24 Stunden)

Während insgesamt 28 Anwendungen erfolgte außerdem gleichzeitig eine topische Antibiotika-Therapie (Creme-Behandlung), während 13 Anwendungen ohne gleichzeitige Antibiotikatherapie durchgeführt wurden.

Insgesamt zeigte sich jedoch, dass die beobachteten Verbesserungseffekte sowohl bei Anwendungen mit gleichzeitiger Antibiotikagabe als auch bei solchen ohne Antibiotikabehandlung auftraten. Umgekehrt zeigten sich auch bei Matricol-Anwendungen mit gleichzeitiger Antibiotikatherapie keine Veränderungen des Hautzustandes durch die Anwendung (Ergebnisse nicht im Einzelnen dargestellt), Damit können die deutlich erzielten Verbesserungen des Hautzustandes nach der Matricol-Anwendung nicht zwangsläufig der Antibiotika-Behandlung zugeschrieben werden sondern müssen vielmehr als davon unabhängig betrachtet werden.

Die folgende Tabelle zeigt die untersuchten Beurteilungsparameter und gibt für jeden Parameter jeweils die Gesamtanzahl an Beurteilungen aus den insgesamt 41 Einzelanwendungen an. Dabei wird unterschieden zwischen Patienten mit leichtem Erkrankungsgrad und schwerem Erkrankungsgrad. Desweiteren werden die Ergebnisse gemäß der Beurteilung der Patienten 15 Minuten nach der Anwendung sowie nach 24 Stunden sowie die Beurteilung des Prüfarztes 15 Minuten nach Anwendung einander gegenübergestellt. Dabei kennzeichnet (+) eine Verbesserung und (-) zeigt einen unveränderten Zustand an.

| Beurteilung | Abnahme der Rötung | | HautBeruhigung | | Angenehmes Hautgefühl | | Normallslerung der Durchblutung | | Ausprägung der Teleanglektasie | | Reduzierung von Brennen, Jucken, Kribbeln, Taubheit | |
|---|---|---|---|---|---|---|---|---|---|---|---|---|
| | + | - | + | - | + | - | + | - | + | - | + | - |
| Patient nach 15 Minuten | | | | | | | | | | | | |
| Leichte Ausprägung | 16 | 5 | 17 | 1 | 20 | 1 | 17 | 4 | | | | |
| Schwere Ausprägung | 19 | 1 | 12 | 2 | 20 | 0 | 17 | 3 | | | | |
| | | | | | | | | | | | | |
| Arzt nach 15 Minuten | | | | | | | | | | | | |
| Leichte Ausprägung | 16 | 5 | 17 | 1 | 21 | 0 | 17 | 4 | 15 | 6 | | |
| Schwere Ausprägung | 19 | 1 | 18 | 2 | 20 | 0 | 18 | 2 | 17 | 3 | | |
| | | | | | | | | | | | | |
| Patient nach 24 Stunden | | | | | | | | | | | | |
| Leichte Ausprägung | 9 | 8 | 11 | 4 | 14 | 3 | 12 | 5 | | | 11 | 5 |
| Schwere Ausprägung | 17 | 3 | 17 | 3 | 19 | 1 | 17 | 3 | | | 17 | 4 |

### Beispiel 3

### Verwendung von gefriergetrockneten schichtförmigen Collagenmatrices in der Behandlung von induzierten entzündlichen Hauterkrankungen (Läsionen) in Folge einer lokalen Behandlung aktinischer Keratose mit Immunstimulanzien

In Folge einer 4-wöchentlichen Behandlung (jeweils Montag, Mittwoch und Freitag) einer aktinischen Keratose im Gesicht, oberhalb der Augenbraue unter dem Haaransatz, mit Aldara (Wirkstoff Imiquimod), einer Substanz, die immunstimulierend wirkt, kam es zu erwünschten entzündlichen Reaktionen an und um die betroffene Hautstelle.

Betroffen war ein Areal von ca. 2 - 3 cm Durchmesser.

Die betroffene Hautstelle war geschwollen, stark gerötet und sehr schmerzempfindlich.

Nach Beendigung der Behandlung wurde die aufgetretene Entzündungsreaktion zunächst durch Auftragen (2 x täglich) von Pyolysinsalbe (Wirkstoffkombination: Pyolysin + Zinkoxid + Salicylsäure) behandelt. Dadurch konnte jedoch keine Verbesserung der Entzündungsreaktionen erzielt werden.

Es wurde anschließend eine Behandlung mit schichtförmigen gefriergetrockneten Collagenmatrices mit einem Collagengehalt > 90 Gew.-% vorgenommen.

Für die Behandlung wurden die schichtförmigen Collagenmaterialien auf Defektgröße zugeschnitten, mit Wasser befeuchtei und für ca. 20 min auf die betroffene Hautstelle aufgelegt.

Zu Beginn der Behandlung wurden die Collagenmaterialien zweimal unmittelbar hintereinander aufgelegt, so dass die betroffene Hautstelle insgesamt für ca, 40 min mit dem Collagenmaterial bedeckt war.

Bereits nach der 2. Anwendung am ersten Tag zeigte sich an der betroffenen Stelle eine deutliche Verbesserung der Schmerz- und Druckempfindlichkeit sowie ein Nachlassen des Stechens der gereizten Haut. Dieser Effekt blieb auch nach Entfernung der Collagenauflage für ca. 2h erhalten.

Die Behandlung wurde eine weitere Woche 1 x täglich, nach Reinigung der betroffenen Hautstelle mit warmem Wasser, fortgeführt. Nach Entfernung der Collagenauflage wurde jeweils eine herkömmliche Feuchtigkeitspflege auf dem gesamten Gesicht aufgetragen.

### Ergebnis der Behandlung:

Abgesehen von dem angenehm kühlenden Effekt zeigte sich eine sehr schnelle entzündungshemmende und abschwellende, und damit verbunden eine schnelle schmerzlindernde Wirkung.

Nach 1-wöchentlicher Anwendung war die betroffene Hautstelle nicht mehr erhaben und gerötet. Eine verbleibende Verfärbung der Haut, die erfahrungsgemäß durch die Beseitigung der Keratose auftritt, glich sich allmählich der übrigen Hautfarbe wieder an.

Vergleichende Behandlungen ähnlicher Defekte im Gesicht an der Schläfe in Folge der Behandlung von aktinischer Keratose mit Aldara unter ausschließlicher Anwendung von Pyolysinsalbe konnten ein vergleichbares Behandlungsergebnis frühestens nach einer Behandlungszeit von etwa 1 Monat erzielen.

### Beispiel 4

### Verwendung von gefriergetrockneten schichtförmigen Collagenmatrices in der Behandlung von allergischen Hautreaktionen mit starker Rötung und Überwärmung des Gewebes, starkem Juckreiz und Quaddelbildung in Folge einer systemischen Antibiotikatherapie

In Folge einer 12-tägigen systemischen (oralen) Antibiotikatherapie traten bei einer weiblichen Patientin (35 Jahre) lokale allergische Hautreaktionen in Form von starker Rötung und Überwärmung des Gewebes, starkem Juckreiz und Quaddelbildung in Dekolletée und Halsbereich auf.

In einem Halbseitenversuch wurde auf die betroffenen Hautareale einerseits eine mit Wasser befeuchtete schichtförmige gefriergetrocknete Collagenmatrix der Marke Matricol® mit einem Collagengehalt > 90 Gew.-% und andererseits eine mit Wasser befeuchtete herkömmliche Baumwoll- bzw. Mull-Verbandskompresse aufgelegt. Die Anwendungsdauer betrug ca. 20 Minuten.

Die beiden Materialien wurden hinsichtlich der Anwendbarkeit und Handhabung, Tragegefühl sowie insbesondere deren Einfluss auf die vorliegenden Symptome und Beschwerden verglichen.

### Ergebnis der Behandlung:

Die gefriergetrocknete Collagenmatrix zeichnete sich durch eine deutlich einfachere und leichtere Handhabung aus. Durch die hohe Anschmiegsamkeit und Elastizität des befeuchteten Materials ließ es sich besonders gut auf die betroffenen Hautareale aufbringen und blieb auch bei Bewegung und in aufrechter Körperhaltung optimal platziert und.

Im Gegensatz dazu zeigte die Mullkompresse deutlich schlechtere Haftungs- und Appliziereigenschaften.

Die Collagenmatrix wies darüber hinaus über den gesamten Anwendungszeitraum eine hohe Feuchtigkeit auf, wohingegen die Mullkompresse nach kurzer Zeit bereits trocknete, was dann auch dazu führte, dass die trocknende Kompresse in aufrechter Körperhaltung nicht mehr auf dem Behandlungsareal verblieb.

Besonders auffällig war der extrem hohe Kühlungseffekt der befeuchteten Collagenmatrix und die deutliche Reduzierung des Juckreizes. Dieser vorteilhafte Effekt hielt insbesondere auch nach der Anwendung noch für mindestens 60 Minuten an, Ein solcher Effekt konnte mit der feuchten Mullkompresse nicht annähernd erzielt werden.

Die mit der Collagenmatrix behandelte Haut fühlte sich außerdem deutlich weicher und elastischer an als die übrigen Hautbereiche.

## Patentansprüche

1. Collagen, welches ausgewählt ist aus der Gruppe der tierischen Collagene, vorzugsweise bovines, porcines oder equines Collagen, zur Verwendung in der Behandlung entzündlicher und degenerativer Hauterkrankungen und damit verbundener Hautschädigungen.

2. Collagen zur Verwendung gemäß Anspruch 1, welches die folgenden Fraktionen umfasst:
a) faserförmig unlösliches Collagen
b) nativ-lösliches (säurelösliches) Collagen
c) Collagen-Peptide.

3. Collagen zur Verwendung gemäß einem der vorhergehenden Ansprüche, welches in Form einer Collagenzubereitung vorliegt, die außerdem mindestens einen Stoff aus der Gruppe der Wirk- und Hilfsstoffe umfasst.

4. Collagen zur Verwendung gemäß Anspruch 3, worin mindestens ein Wirkstoff enthalten ist, der ausgewählt ist aus der Gruppe der hautähnlichen Lipide und/oder aus der Gruppe der Substanzen des Natural Moisturizing Factors (NMF).

5. Collagen zur Verwendung gemäß einem der vorhergehenden Ansprüche, welches in Form von schichtförmigen Auflagen, Sheets, Pads oder Masken vorliegt.

6. Collagen zur Verwendung gemäß einem der vorhergehenden Ansprüche, welches gefriergetrocknet vorliegt.

7. Collagen zur Verwendung gemäß Anspruch 5 oder 6, welches zu mindestens 50 Gew.-% aus Collagen besteht.

8. Collagen zur Verwendung gemäß einem der Ansprüche 5 bis 7, welches eine zusätzliche Schicht aus Fasern, Vliesen, Netzen, Filmen oder Folien oder eine selbstklebende Schicht aufweist, wobei diese zusätzliche Schicht das Collagenmaterial teilweise oder vollständig bedeckt und so auf dem schichtförmigen Collagenmaterial aufgebracht ist, dass es mit diesem randseitig abschließt oder randseitig vollständig oder partiell über das Collagen material hinausragt.

9. Collagen zur Verwendung gemäß einem der vorhergehenden Ansprüche, worin die Hauterkrankungen oder Hautschädigungen **gekennzeichnet sind durch** Spannung, Rötung, Hitze, Pruritus (Juckreiz), Schuppung sowie ekzematöse Veränderungen auch in Form von Blasenbildung, Quaddeln oder Pusteln.

10. Collagen zur Verwendung gemäß einem der vorhergehenden Ansprüche, umfassend die Behandlung von Psoriasis, Dermatitis, Neurodermitis, atopischer Dermatitis, Rosacea, Urticaria (Nesselsucht), Pruritus (Juckreiz), Hautekzemen bzw, ekzematösen Veränderungen, Erythema und aktinischer Keratose sowie entzündlichen Läsionen in Folge einer lokalen Behandlung aktinischer Keratose mit Immunstimulanzien, sowie jeweils der damit einhergehenden Symptome.

11. Collagen zur Verwendung gemäß einem der vorhergehenden Ansprüche, worin die Verwendung die folgenden Behandlungs-Schritte umfasst:
a) Bereitstellen einer schichtförmigen, trockenen Applikationsform aus Collagen oder einer Collagenzubereitung
b1) Befeuchten des trockenen schichtförmigen Collagenmaterials mit einer Aktivatorlösung, die ausgewählt ist aus Wasser, physiologischen Lösungen sowie wässrigen Lösungen die mindestens einen Wirk- und/oder Hilfsstoff enthalten und Aufbringen des befeuchteten Collagenmaterials auf die zu behandelnden Hautareale
oder
b2) Aufbringen des trockenen schichtförmigen Collagenmaterials auf die zu behandelnden Hautareale und Befeuchten des trockenen schichtförmigen Collagenmaterials auf den zu behandelnden Hautarealen mit einer Aktivatorlösung, die ausgewählt ist aus Wasser, physiologischen Lösungen sowie wässrigen Lösungen die mindestens einen Wirk- und/oder Hilfsstoff enthalten.

12. Collagen zur Verwendung gemäß Anspruch 11 worin die befeuchteten schichtförmigen Collagenmaterialien bis zu 60 Minuten auf dem zu behandelnden Hautareal verbleiben.

13. Collagen zur Verwendung gemäß einem der vorhergehenden Ansprüche, worin die Anwendung topisch erfolgt.

14. Kit-of-parts Kombination enthaltend Collagen zur Verwendung gemäß einem der vorhergehenden Ansprüche sowie eine wässrige Lösung, die ausgewählt ist aus Wasser, physiologischen Lösungen sowie wässrigen Lösungen die mindestens einen Wirk- und/oder Hilfsstoff enthalten, in zusammengehöriger räumlicher Anordnung.

## Claims

1. Collagen, which is selected from the group of animal collagens, preferably bovine, porcine or equine collagen, for use in the treatment of Inflammatory and degenerative skin diseases and damage to the skin connected therewith.

2. Collagen for the use according to claim 1, comprising the following fractions:
a) fibrous insoluble collagen
b) natively soluble (acid-soluble) collagen
c) collagen peptides.

3. Collagen for the use according to any one of the preceding claims, provided in the form of a collagen preparation which In addition comprises at least one substance from the group of active or auxiliary substances.

4. Collagen for the use according to claim 3, wherein at least one active substance is contained that is selected from the group of the skin-like lipids and/or from the group of substances of the natural moisturizing factor (NMF).

5. Collagen for the use according to any one of the preceding claims, provided In the form of layered dressings, sheets, pads or masks.

6. Collagen for the use according to any one of the preceding claims, provided in a freeze-dried form.

7. Collagen for the use according to claim 5 or 6, which consists of at least 50% by wt. collagen.

8. Collagen for the use according to any one of the claims 5 to 7, comprising an additional layer of fibers, non-wovens, nets, films or folls or an adhesive layer, with this additional layer covering the collagen material partially or completely and being applied on the layered collagen material in such a way that It ends flush with the edges thereof or protrudes completely or partially over the collagen material at the edges.

9. Collagen for the use according to any one of the preceding claims, wherein the skin diseases or damage to the skin are **characterized by** tightness, erythema, heat, pruritus (itching), scaling as well as eczematous changes also in the form of the formation of blisters, wheals or pustules.

10. Collagen for the use according to any one of the preceding claims, comprising the treatment of psoriasis, dermatitis, neurodermitis, atopical dermatitis, rosacea, urticaria (hives), pruritus (itching), skin eczema or eczematous changes, erythema and actinic keratosis as well as inflammatory lesions as a consequence of a local treatment of actinic keratosis with immunostimulants, as well as, respectively, the symptoms associated therewith.

11. Collagen for the use according to any one of the preceding claims, wherein the use comprises the following treatment steps:
a) providing a layered dry form of application consisting of collagen or a collagen preparation
b1) moistening the dry layered collagen material with an activator solution selected from water, physiological solutions as well as aqueous solutions containing at least one active and/or auxiliary substance, and applying the moistened collagen material to the skin areas to be treated
or
b2) applying the dry layered collagen material to the skin areas to be treated, and moistening the dry layered collagen material on the skin areas to be treated with an activator solution selected from water, physiological solutions as well as aqueous solutions containing at least one active and/or auxiliary substance.

12. Collagen for use according to claim 11, wherein the moistened layered collagen materials remain on the skin area to be treated for up to 60 minutes.

13. Collagen for use according to any one of the preceding claims, wherein the application is carried out topically.

14. Kit-of-parts combination, comprising collagen for the use according to any one of the preceding claims as well as an aqueous solution selected from water, physiological solutions as well as aqueous solutions containing at least one active and/or auxiliary substance, In an associated spatial arrangement.

## Revendications

1. Collagène qui est sélectionné parmi le groupe des collagènes d'animaux, préférablement le collagène bovin, porcin ou équin, pour l'utilisation dans le traitement des maladies inflammatoires et dégénératives de la peau et des lésions de la peau y associées.

2. Collagène pour l'utilisation selon la revendication 1, qui comprend les fractions suivantes :
a) du collagène fibreux insoluble
b) du collagène nativement insoluble (soluble dans l'acide)
c) des peptides de collagène.

3. Collagène pour l'utilisation selon l'une des revendications précédentes, qui est en forme d'une préparation de collagène, qui comprend en outre au moins une substance du groupe des agents actifs et des agents excipients.

4. Collagène pour l'utilisation selon la revendication 3, dans lequel au moins un agent actif est contenu, qui est sélectionné parmi le groupe des lipides semblables à la peau et/ou parmi le groupe des substances du facteur naturel d'hydratation (FNH).

5. Collagène pour l'utilisation selon l'une des revendications précédentes, qui est lyophilisé.

6. Collagène pour l'utilisation selon l'une des revendications précédentes, qui est lyophilisé.

7. Collagène pour l'utilisation selon la revendication 5 ou 6, qui est composé de 50 % en poids de moins de collagène.

8. Collagène pour l'utilisation selon l'une des revendications 5 à 7, qui a une couche additionnelle de fibres, de nappes, de films ou de feuilles ou une couche autoadhésive, ladite couche autoadhésive couvrant le matériau de collagène de façon partielle ou complète et étant appliquée sur le matériau de collagène feuilleté tel qu'elle le ferme en bordure ou est latéralement en saille par rapport du matériau de collagène de façon partielle ou complète.

9. Collagène pour l'utilisation selon l'une des revendications précédentes, dans lequel les maladies de la peau ou les lésions de la peau sont **caractérisées par** la tension, le rougeur, la chaleur, la prurit (les démangeaisons), la desquamation ainsi que des changements eczémateux, également en forme de formation de cloques, de papules ou de pustules.

10. Collagène pour l'utilisation selon l'une des revendications précédentes, comprenant le traitement de la psoriasis, la dermatite, la névrodermite, la dermatite atopique, la rosacée, l'urticaire (les ruches), la prurit (les démangeaisons), les eczèmes de la peau et les changements eczémateux, l'érythème et la kératose actinique ainsi que les lésions inflammatoires à la suite d'un traitement local de la kératose actinique avec des agent immuno-stimulatifs, ainsi que chacun des symptômes y associés.

11. Collagène pour l'utilisation selon l'une des revendications précédentes, dans lequel l'utilisation comprend les séquences de traitement suivantes consistant à :
a) fournir une forme d'application feuilletée et sèche de collagène ou une préparation de collagène
b1) humidifier le matériau feuilleté sec avec une solution activatrice, qui est sélectionnée de l'eau, les solutions physiologiques ainsi que les solutions aqueuses qui contiennent au moins un agent actif et/ou un agent excipient, et appliquer le matériau de collagène humidifié sur les régions de la peau à traiter
ou
b2) appliquer le matériau feuilleté sec sur les régions de la peau à traiter et humidifier le matériau feuilleté sec sur les régions de la peau à traiter avec une solution activatrice, qui est sélectionnée de l'eau, les solutions physiologiques ainsi que les solutions aqueuses qui contiennent au moins un agent actif et/ou un agent excipient.

12. Collagène pour l'utilisation selon la revendication 11, dans lequel les matériaux de collagène feuilletés humidifiés restent sur les régions de la peau à traiter jusqu'à 60 minutes.

13. Collagène pour l'utilisation selon l'une des revendications précédentes, dans lequel l'application est faite topiquement.

14. Combinaison de compositions pharmaceutiques ou collection (combinaison de « kit of parts ») contenant du collagène pour l'utilisation selon l'une des revendications précédentes, ainsi qu'une solution aqueuses, qui est sélectionnée parmi l'eau, les solutions physiologiques ainsi que les solutions contenant au moins un agent actif ou un agent excipient, dans un arrangement spatial associé.
